# EUROPEAN PATENT APPLICATION

(11) **EP 1 186 350 A1**
(43) Date of publication of application: **13.03.2002**
(21) Application number: 00922911.3
(22) Date of filing: 28.04.2000
(51) Int. Cl.: B05B 17/00, B05B 17/06, A61M 11/00

(54) **LIQUID SPRAY DEVICE**

(30) Priority: 28.04.1999 JP 12132399
(71) Applicant: Omron Corporation, Kyoto-shi, Kyoto-fu, 600-8530 (JP)
(72) Inventor: TANAKA, Shinya, Omron Corporation, Kyoto-shi, Kyoto 616-8025 (JP); ASAI, Kei, Omron Corporation, Kyoto-shi, Kyoto 616-8025 (JP); ITOH, Shin-ichi, Omron Corporation, Kyoto-shi, Kyoto 616-8025 (JP); ARAI, Masato, Omron Corporation, Kyoto-shi, Kyoto 616-8025 (JP)
(74) Representative: WILHELMS, KILIAN & PARTNER Patentanwälte
(86) International application number: JP0002837
(87) International publication number: WO0066277

(57) **Abstract**

An atomizing chamber 10C is provided in a part of a device housing 1, and a liquid feeding mechanism 30 is provided in the other part of the device housing. At least a part of ultrasonic vibrators 20, 21 is disposed inside the atomizing chamber, and a liquid feeding hole 16a is provided in part of a wall of the atomizing chamber. A liquid bottle 90 having a minute hole provided at a tip end thereof and having a movable piston 93 provided therein is mountable on one end of the liquid feeding mechanism, and the liquid feeding mechanism can be positioned such that the minute hole of the liquid bottle mounted on the liquid feeding mechanism and the liquid feeding hole formed in the atomizing chamber coincide with each other. The liquid feeding mechanism has a manual operating member 40 and pressing mechanisms 50, 60 for causing the piston in the mounted liquid bottle to advance by a predetermined stroke when the manual operating member is operated.

## Description

### Technical Field

The present invention relates to a device for atomizing a liquid (including an aqueous medication) by ultrasonic vibration, which is called by an ultrasonic atomizer (nebuliger), an ultrasonic inhaler and other names, a liquid feeding (supplying) mechanism, an atomizing mechanism, or an atomization controller which constitutes a part of the atomizing device, and a liquid bottle (a aqueous medication ampoule) used for supplying a liquid (mainly a aqueous medication) to a user with the bottle filled with the liquid and having a structure particularly suitable for the atomizing device.

### Background Art

In an electric inhaler or a portable inhaler which is used by a user (a patient) himself or herself, it is difficult to prescribe a dose of aqueous medication to be used correctly and sanitarily.

JP-A-8-502689 describes various examples of feeding devices. Also in the examples, a user measures the amount of feed of a liquid, or the structure of a measuring device is not fully disclosed.

### Disclosure of Invention

An object of the present invention is to provide a liquid feeding mechanism (supplying device) capable of always feeding a fixed amount of liquid in simple operations.

Another object of the present invention is to provide a liquid bottle suitable for the above-mentioned liquid feeding mechanism (device).

Still another object of the present invention is to provide a mechanism (device) capable of atomizing a fed liquid without leaving the liquid as possible.

A further object of the present invention is to provide a liquid atomizer (liquid atomizing device) having the above-mentioned features.

A still further object of the present invention is to provide a liquid atomizer (liquid atomizing device) suitable for portability.

A liquid feeding mechanism capable of always feeding a fixed amount of liquid according to the present invention comprises a cylindrical housing having an end on which a liquid bottle having a movable piston disposed therein is mountable; a pushing operating member which is pressed (by a manual operation) to be moved by more than a first predetermined stroke (which may be the first predetermined stroke) and is returned to its original position (when there is no pressing force by the manual operation); a fixed angle rotating mechanism for converting movement by more than the first stroke of the pushing operating member into forward rotation of a predetermined angle; and a screw which is held in the cylindrical housing so as to be rotatable by screwing and is disposed so as to advance by a second predetermined stroke when it is rotated in a forward direction through the predetermined angle by the fixed angle rotating mechanism to press the piston in the liquid bottle. The liquid bottle includes an aqueous medication bottle or a aqueous medication ampoule (ampul or ampule). Generally, a hole (a minute hole) is provided at a tip end of the liquid bottle, or a valve is provided in addition to the hole.

A user may press the pushing operating member. Motion by a user's pushing (or pressing) operation is converted into rotation of a predetermined angle. The rotation of the predetermined angle is converted into motion by a predetermined stroke of the screw for pressing the piston in the liquid bottle. The piston in the liquid bottle is always pressed by the predetermined stroke. If the cross-sectional area of the liquid bottle is constant along the length thereof, a fixed amount of liquid is always jetted or ejected from the liquid bottle.

Preferably, the first stroke is longer than the second stroke. Even when a small amount of liquid is fed (supplied), therefore, the user's operation is simple.

Preferably, there is provided a stopper for regulating the amount of movement of the pushing operating member. The user's operation becomes simple.

Preferably, a portion projecting outward is provided at a tip end of the screw, and a tip end of this portion is so formed as to be rounded. The rotation of the screw does not adversely affect the piston.

In a desirable embodiment, there is further provided a returning mechanism which engages with the fixed angle rotating mechanism for rotating the fixed angle rotating mechanism in a reverse direction to cause the screw to retreat. The screw is caused to retreat, thereby making it easy to replace the liquid bottle.

More specifically, the pushing operating member comprises a knob guiding member which is provided so as to be rotatable at the other end of the cylindrical housing, a knob shaft which is provided so as to be axially movable in a state where the rotation thereof is prevented by the knob guiding member, and a knob which is provided at an outer end of the knob shaft.

The fixed angle rotating mechanism comprises a fixed angle rotating member which engages with the screw so as to be rotated together with the screw and so as to be movable along the axis direction of the screw, a sliding finger which is fixed to the fixed angle rotating member, extends along the knob shaft, and has a slope formed at a tip end thereof, a return spring for urging the fixed angle rotating member toward the knob guide, a cam cylinder which is provided inside the knob guiding member, and has an oblique cam surface which the tip end of the sliding finger brought into contact with and slides by a force of said return spring to rotate the fixed angle rotating member, and a pressing member which is provided at an inner end of the knob shaft, and has a cam surface for pressing the sliding finger against the force of the return spring in contact with the slope at the tip end of the sliding finger when the knob shaft is pressed inward through the knob to release the engagement of the sliding finger with the cam cylinder and slightly rotating the fixed angle rotating member to put the tip end of the sliding finger on the subsequent oblique cam surface of the cam cylinder.

The returning member comprises the knob guiding member. The knob guiding member is of a cylindrical shape and is provided so as to cover the other end portion of the cylindrical housing. An annular groove and a lock groove connecting therewith are formed on a peripheral surface of the other end portion of the cylindrical housing, an inner end of a lock pin provided so as to penetrate a peripheral wall of the knob guiding member enters the annular groove or the lock groove so as to be movable, and a lock spring for urging the knob guiding member outward is provided between the other end of the cylindrical housing and the knob guiding member.

More generally expressed, the liquid feeding mechanism according to the present invention comprises a cylindrical housing having an end on which a liquid bottle having a movable piston disposed therein is mountable; an operating member which is moved by a manual operation and is returned to its original position; a fixed angle rotating mechanism for converting movement of the operating member into forward rotation of a predetermined angle; and a screw which is held in the cylindrical housing so as to be rotatable by screwing and is disposed so as to advance by a predetermined stroke when it is rotated in a forward direction through the predetermined angle by the fixed angle rotating mechanism to press the piston in the liquid bottle.

A liquid bottle according to the present invention which is suitable for the liquid feeding mechanism has a minute hole formed at a tip end thereof, opens at a distal end thereof, has a movable piston disposed therein, and is provided with a catch used to mount the liquid bottle on a liquid feeding mechanism for pressing the piston and for resisting a force for pressing the piston. The catch may be a come-off preventing portion against a force for pressing the piston, which may be a recess, a projection, or a slip preventing portion.

It is preferable that when the piston is formed of an elastic member, a hard receiving member which receives a pressing force from the liquid feeding mechanism is provided in the piston.

A cap for covering the tip end including the minute hole is provided, so that the liquid bottle becomes more sanitary.

An atomizing mechanism according to the present invention which is improved to atomize a fed liquid without leaving the liquid as possible, comprises an atomizing chamber in which at least a part of an ultrasonic vibrator is provided, wherein a liquid feeding hole is formed on a wall of the atomizing chamber, a liquid feeding pipe is provided from the liquid feeding hole toward the inside of the atomizing chamber, a tip end of the liquid feeding pipe reaches the vicinity of the ultrasonic vibrator, and a liquid buffer exists in a part of the liquid feeding hole or the liquid feeding pipe.

The liquid feeding pipe may be a hole communicating with the liquid feeding hole and formed in a projection formed integrally with the wall of the atomizing chamber so as to project toward the inside of the atomizing chamber.

The liquid buffer may be a clearance between a tip end of the liquid bottle having the minute hole for feeding the liquid into the atomizing chamber through the liquid feeding hole formed therein and an outer surface of the wall of the atomizing chamber, or may be a liquid reservoir formed halfway in the liquid feeding hole or the liquid feeding pipe and opening toward the air.

The liquid is fed into the vicinity of the ultrasonic vibrator through the liquid feeding pipe, so that almost all of the liquid is atomized. The liquid buffer exists halfway. Accordingly, the liquid is also stored in this buffer portion, and sucks in as the atomization progresses. The liquid which is not fed into the vicinity of the ultrasonic vibrator is also atomized.

When a tip end of the liquid feeding pipe is obliquely cut, the liquid is easily held.

In one embodiment, the ultrasonic vibrator comprises a horn and a mesh plate provided on an upper end surface of the horn.

An atomizing mechanism having the above-mentioned features according to the present invention can be expressed as follows. That is, the atomizing mechanism comprises an atomizing casing having a cylindrical atomizing chamber having a hole formed on its lower surface; a horn, a part of which projects into the atomizing chamber from the hole of the casing, driven by ultrasonic vibration; a mesh plate disposed on an upper end surface of the horn; a pressing spring for urging the mesh plate in the direction in which the mesh plate is brought into contact with the upper end surface of the horn; a liquid feeding pipe penetrating the wall of the atomizing chamber, and introduced into the vicinity of the upper end of the horn; and a plate placed on a bottom surface of the atomizing chamber and having a hole formed therein having such an inner diameter as to hold a clearance between the hole and a peripheral surface of the horn. When the amount of liquid to be fed is small, the plate is not necessarily required.

In all the atomizing mechanisms described above, it is further desirable to provide a driving control circuit for stopping the driving of the ultrasonic vibrator once at the time point where the atomization of the liquid fed into the atomizing chamber through the liquid feeding pipe is almost terminated, and then driving the ultrasonic vibrator in a pulse shape once or intermittently a plurality of times in a short time. The remaining liquid is atomized by temporary stop and pulse-shaped ultrasonic vibration subsequent thereto. The amount of liquid which remains without being atomized is further reduced.

When the ultrasonic vibrator is more strongly vibrated in the short time, the effect is further enhanced.

In a device for feeding a predetermined amount of liquid to a ultrasonic vibrator to atomize the liquid by the vibration of the ultrasonic vibrator, an atomization controller according to the present invention for making the amount of liquid which remains without being atomized as small as possible comprises means for detecting the time point where the atomization of the liquid fed into the ultrasonic vibrator is almost terminated ( including measurement of time from the start of the atomization); and first control means for carrying out control so as to stop the driving of the ultrasonic vibrator once in response to the detection of the time point where the atomization of the liquid is almost terminated and to drive the ultrasonic vibrator in a pulse shape once or intermittently a plurality of times in a short time after an elapse of a short time since the driving was stopped.

The atomization controller preferably further comprises means for detecting the feed of the liquid to the ultrasonic vibrator in a state where the ultrasonic vibrator is weakly vibrated (which may be means responding to the manual operation switch) , and second control means for carrying out control so as to drive the ultrasonic vibrator by such normal power that the liquid is atomized in response to the detection of the feed of the liquid.

In a preferred embodiment, the first control means carries out control so as to drive the ultrasonic vibrator more strongly than the second control means.

In another embodiment, there is further provided third control means for carrying out control so as to weakly vibrate the ultrasonic vibrator in response to the turn-on of a power switch.

The second control means may cause the ultrasonic vibrator to be driven by such normal power that the liquid can be atomized in response to the power switch or a start switch.

In a liquid atomizer according to the present invention, an atomizing chamber is provided in a part of a device housing, a liquid feeding mechanism is provided in the other part of the device housing, at least a part of a ultrasonic vibrator is disposed inside the atomizing chamber, a liquid feeding hole is provided in a part of a wall of the atomizing chamber, a liquid bottle having a minute hole formed at its tip end and having a movable piston provided therein is mountable on one end of the liquid feeding mechanism, the liquid feeding mechanism can be positioned such that the minute hole of the liquid bottle mounted on the liquid feeding mechanism and the liquid feeding hole formed in the atomizing chamber coincide with each other, and the liquid feeding mechanism has a manual operating member and a pressing mechanism for causing the piston in the mounted liquid bottle by a predetermined stroke when the manual operating member is operated. The device is particularly advantageous for a portable type for personal use.

In a preferred embodiment, a small clearance exists between the tip end, at which the minute hole is formed, of the liquid bottle and an outer surface of the wall, on which the liquid feeding hole is formed, of the atomizing chamber at a position where the minute hole of the liquid bottle and the liquid feeding hole of the atomizing chamber coincide with each other.

In another embodiment, the ultrasonic vibrator comprises an ultrasonic vibrator horn at least a part of which is disposed in the atomizing chamber and a mesh plate disposed so as to be brought into contact with an upper end of the horn by being pressed by a spring, the liquid feeding pipe extends from the liquid feeding hole toward the vicinity of the upper end portion of the horn.

Desirably, the tip end of the liquid feeding pipe is obliquely cut.

Preferably, the device housing is provided with a mounting member for detachably mounting the liquid feeding mechanism. It is preferable that the mounting member is movable.

In another embodiment, the device housing is provided with a positioning member for the liquid feeding mechanism.

Desirably, the device housing is provided with a cover for covering at least its part provided with the atomizing chamber.

In one embodiment, the liquid feeding mechanism comprises a fixing member for fixing the liquid bottle.

In one embodiment, the liquid feeding mechanism comprises a cylindrical housing having an end on which the liquid bottle is mountable . The manual operating member is moved by a manual operation and is returned to its original position. The pressing mechanism comprises a fixed angle rotating mechanism for converting movement of the manual operating member into forward rotation of a predetermined angle, and a screw which is held in the cylindrical housing so as to be rotatable by screwing and is disposed so as to advance by a predetermined stroke when it is rotated in a forward direction through the predetermined angle by the fixed angle rotating mechanism to press the piston in the liquid bottle. Desirably, the liquid feeding mechanism further comprises a returning mechanism which engages with the fixed angle rotating mechanism and rotates in a reverse direction the fixed angle rotating mechanism to cause the screw to retreat.

In one embodiment, the device housing has a driving control circuit provided therein for stopping the driving of the ultrasonic vibrator once at the time point where the atomization of the liquid fed into the atomizing chamber through the liquid feeding pipe is almost terminated, and then driving the ultrasonic vibrator once or intermittently a plurality of times in a short time.

Alternatively, there is provided in the device housing a controller comprising means for detecting the time point where the atomization of the liquid fed into the ultrasonic vibrator is almost terminated, and control means for carrying out control so as to stop the driving of the ultrasonic vibrator once in response to the detection of the time point where the atomization of the liquid is almost terminated, and then drive the ultrasonic vibrator once or intermittently a plurality of times in a short time after an elapse of a short time since the driving was stopped.

The present invention further provides an inhaler of still another mode. It is an inhaler using a liquid bottle having a hole provided at its one end, the hole being sealed with an elastic member provided in the bottle, having a movable piston provided at the other end, and filled with a liquid, comprising inner and outer accommodating portions which are fitted to each other so as to be movable, the inner accommodating portion accommodating the liquid bottle so as to be movable, and comprising a hollow needle and a returning member, and the outer accommodating portion comprising a member for pressing the piston in the liquid bottle, the inner accommodating portion being moved when the piston in the liquid bottle accommodated in the inner accommodating portion is pressed by the pressing member manually operated, the needle penetrating the elastic member so that the liquid in the liquid bottle is fed into the ultrasonic vibrator through the needle, and the inner accommodating portion being returned by the returning member when a pressing force of the pressing member is removed.

If the pressing member of the piston is operated, the liquid in the liquid bottle is fed in a predetermined amount.

The present invention further provides an ultrasonic vibrator. That is, in the present invention, in an ultrasonic vibrator comprising a horn in an approximately conical shape, a piezoelectric element provided at the base of the horn, and a resonance plate in a disk shape integrally formed at a tip end of the horn, an end surface at the base of the horn which is provided with the piezoelectric element is in a spherical shape, and the piezoelectric element is provided along the end surface in the spherical shape. Consequently, the vibration of the resonance plate is increased, thereby improving the vibration efficiency.

### Brief Description of Drawings

Fig. 1 is a perspective view showing a state where a liquid atomizer is used.
Fig. 2 is an exploded perspective view of a state where the liquid atomizer is accommodated.
Fig. 3 is a perspective view of a state where the liquid atomizer is accommodated.
Fig. 4 is a perspective view of a state where the liquid atomizer is accommodated.
Fig. 5 is a cross-sectional view showing the whole of the liquid atomizer.
Fig. 6 is an enlarged sectional view of an atomizing mechanism in the liquid atomizer.
Figs. 7 and 8 are enlarged sectional views showing how atomization occurs.
Figs. 9 to 11 are cross-sectional views showing a modified example.
Figs. 12a and 12b are perspective views showing the relationship between a fixed angle rotating member and a screw.
Figs. 13a and 13h are cross-sectional views showing the structure and the operations of a fixed angle rotating mechanism.
Figs. 14a and 14b are perspective views showing a returning mechanism.
Fig. 15 is a circuit diagram of an atomization controller, and Fig. 16 is a waveform diagram showing the operations thereof.
Figs. 17 to 20 are cross-sectional views showing the structures and the operations of a liquid feeding portion and an atomizing portion in an ultrasonic inhaler.
Fig. 21 is a cross-sectional view showing a liquid feeding portion and an atomizing portion in another ultrasonic inhaler.
Fig. 22 is a perspective view showing an ultrasonic inhaler which easily sets and adjusts a aqueous medication corresponding to one prescription.
Figs. 23a and 24a are cross-sectional views showing ultrasonic vibrators, and Figs. 23b and 24b are front views of their resonance plates.

### Best Mode for Carrying Out the Invention

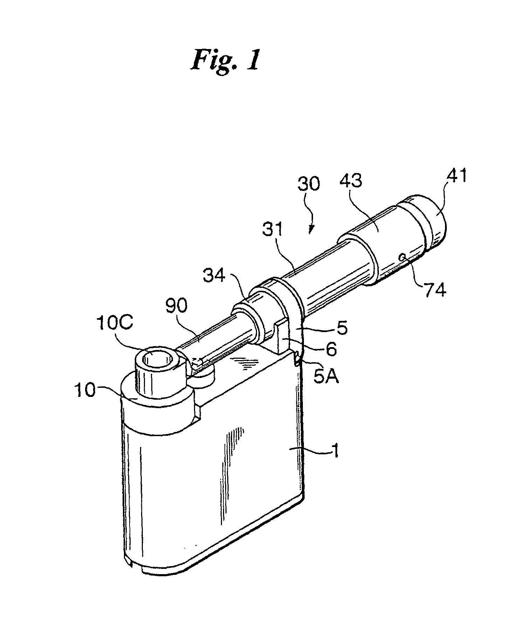

Fig. 1 illustrates a state where a liquid atomizer (liquid atomizing device) in a form suitable for realization of a portable ultrasonic inhaler is used.

A liquid feeding mechanism (or a liquid feeding or supplying device, a fixed amount aqueous medication feeding or supplying device, or a fixed amount (fixed stroke) piston pushing or pressing device) 30 is detachably mounted on a device housing 1. The device housing 1 is provided with a mounting member (a holding member) 5 for mounting the liquid feeding mechanism 30 and a positioning member 6. The mounting member 5 is pivotable in a range of an angle of more than 90° by a pin 5A.

At a position slightly spaced apart from the mounting member 5 and the positioning member 6, the device housing 1 is detachably provided with an atomizing casing 10. The atomizing casing 10 has an atomizing chamber 10C.

An aqueous medication ampoule (a liquid bottle or an aqueous medication bottle) 90 is detachably mounted on a tip end of the liquid feeding mechanism 30 by a fixing member (a fixing ring) 34. A tip end of the aqueous medication ampoule 90 is brought into contact with an outer surface of a wall of the atomizing chamber 10C nearly (spaced a slight clearance apart).

A knob 41 is provided at a rear end of the liquid feeding mechanism 30, and the knob 41 projects outward. A user presses or pushes the knob 41 along the axis of the liquid feeding mechanism 30 with his or her hand. Consequently, an aqueous medication in the aqueous medication ampoule 90 is injected in a predetermined (fixed) amount into the atomizing chamber 10C, and is atomized upward by the vibration of an ultrasonic vibrator disposed in the atomizing chamber 10C.

The user can inhale fine particles of the atomized aqueous medication into his or her nasal cavity or throat, or lungs in some cases. The aqueous medication of a fixed amount is always atomized by pushing the knob 41 once.

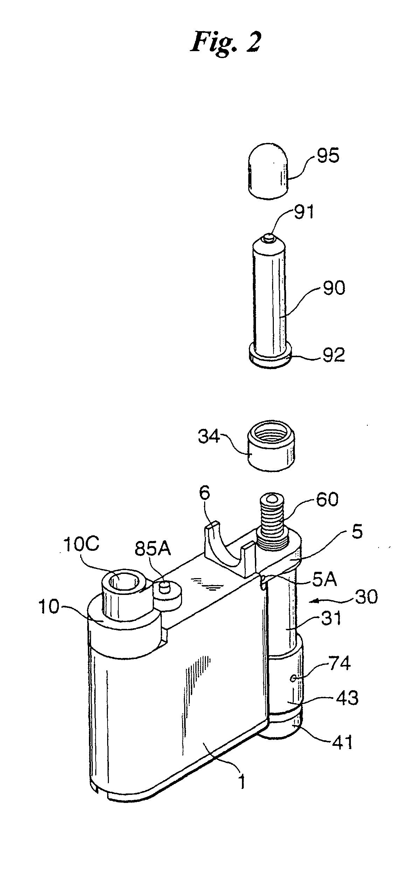

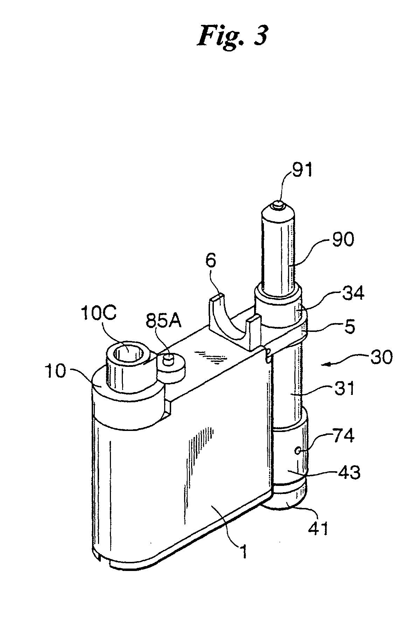

Figs. 2 and 3 illustrate a state where the liquid atomizer is assembled. The aqueous medication ampoule 90 is generally provided separately from the liquid atomizer. The aqueous medication ampoule 90 is a consumer product. Accordingly, the user purchases a required number of aqueous medication ampoules, as required, besides the liquid atomizer.

The aqueous medication ampoule 90 is of a cylindrical shaped, and has a minute (very small) hole 91 provided at its tip end and has a flange 92 for mounting formed at its rear end. As apparent later, a piston is disposed inside the aqueous medication ampoule 90. Preferably, the tip end, at which the minute hole 91 is provided, of the aqueous medication ampoule 90 is covered with a cap 95.

The liquid feeding mechanism 30 comprises a cylindrical housing (a screw holder) 31 which accommodates a screw 60 so as to be rotatable and such that it can advance or retreat. The cylindrical housing 31 is screwed into a male screw formed inside the mounting member 5 so as to be mounted on the device housing 1. The aqueous medication ampoule 90 is mounted on the cylindrical housing 31 with the flange 92 accommodated in the fixing ring 34. The fixing ring 34 is along an arc-shaped inner surface of the positioning member 6. A knob guiding member (or a lock cap or a screw returning member) 43 is provided at a rear end of the cylindrical housing 31, and a lock pin 74 is provided in the knob guiding member 43.

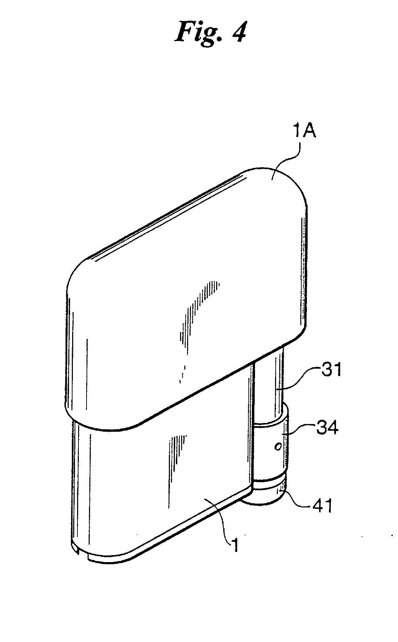

A state, shown in Fig. 3, where the liquid feeding mechanism 30 is along a side surface of the device housing 1 (the liquid feeding mechanism 30 is in a posture which differs by an angle of approximately 90° from that in the used state shown in Fig. 1) (the aqueous medication ampoule 90 may be mounted or may not be mounted) is an accommodated state. In this state, the device housing 1 is covered with a cover 1A (the cover 1 is attachable and detachable), as shown in Fig. 4, thereby making it possible to protect the atomizing chamber 10C and the aqueous medication ampoule 90 or the tip end of the liquid feeding mechanism 30.

A switch button 85A is provided near the atomizing chamber 10C on the upper surface of the device housing 1. When the liquid feeding mechanism 30 is shifted from the accommodated state to the use state shown in Fig. 1, the aqueous medication ampoule 90 presses the switch button 85A. Consequently, the ultrasonic vibrator is driven (weak vibration, as described later), to enter a state where the aqueous medication can be atomized.

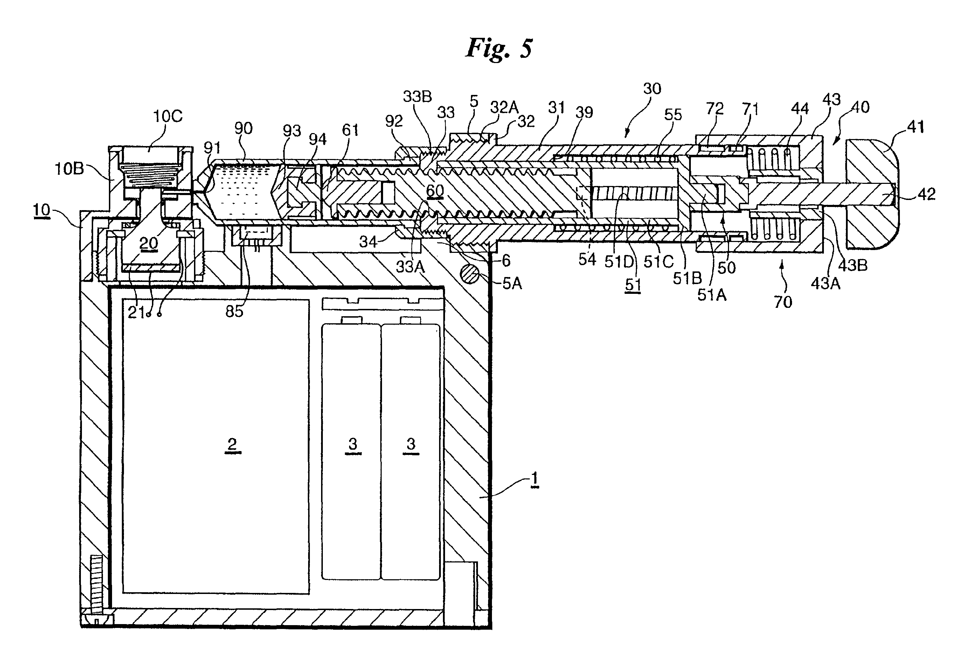

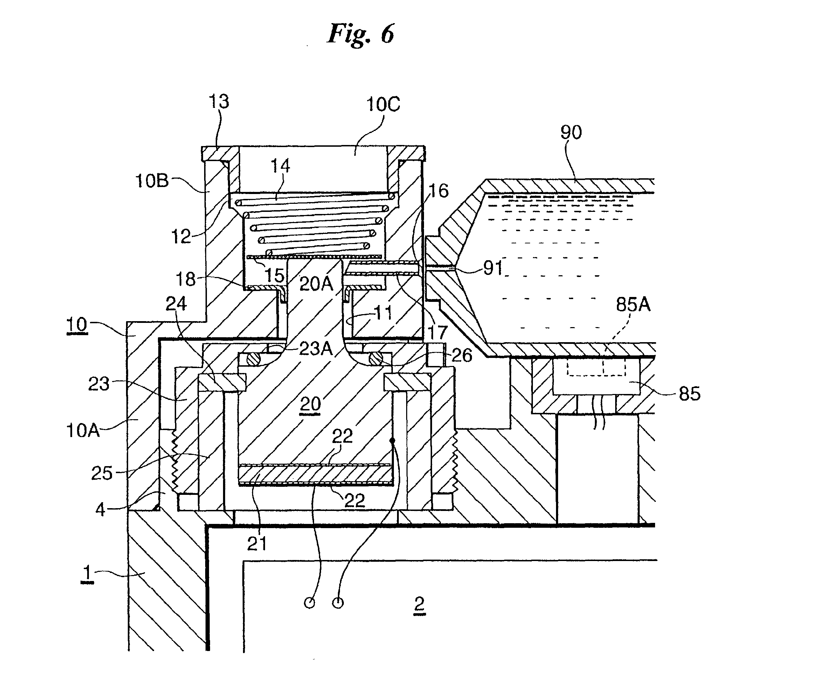

Fig. 5 is a cross-sectional view showing the inside of the whole of the liquid atomizer, and Fig. 6 particularly illustrates in enlarged fashion the details of the inside of the atomizing casing and its vicinity.

The aqueous medication ampoule 90 is basically a cylindrical member made of glass or plastics, and is preferably made of a transparent or translucent material. As previously described, the tip end of the aqueous medication ampoule 90 is tapered in a conical shape, and its tip end surface is flat. The flat tip end surface is provided with the minute hole (an injecting hole, a pouring and feeding hole or a spray hole) 91. The diameter of the minute hole 91 is generally 0.1 mm to 0.5 mm, although it depends on the property of the aqueous medication with which the aqueous medication ampoule 90 is filled. The diameter of the minute hole 91 is preferably 0.2 mm to 0.4 mm, and most preferably 0.3 mm. If the hole has such a diameter, the aqueous medication accommodated into the ampoule 90 does not leak out of the minute hole 91, unless the piston 93 is moved.

The piston 93 is accommodated movably and water-tightly in the aqueous medication ampoule 90. The piston 93 is made of an elastic member such as rubber. A receiving member 94 made of a hard material such as plastics is fitted in a rear surface of the piston 93. The receiving member 94 comprises a plate-shaped portion having a diameter approximately equal to the inner diameter of the aqueous medication ampoule 90. Even if the piston 93 is formed of an elastic member, a pressing (pushing) force by the screw 60 is sufficiently exerted on the piston 93 by the existence of the receiving member 94.

The flange 92 projecting outward is integrally formed at a rear end of the aqueous medication ampoule 90. The fixing ring 34 is formed with a flange projecting inward. The flange 92 of the aqueous medication ampoule 90 is positioned inside the flange of the fixing ring 34. Consequently, the aqueous medication ampoule 90 is prevented from dropping (particularly, by the pushing force by the screw 60). The fixing ring 34 is screwed with a screw 33B formed on an outer surface of the tip end portion (a fitting portion or a screw holding portion) 33 of the cylindrical housing 31 and is fixed thereto.

A portion like the flange 92 of the aqueous medication ampoule 90 is referred to as a catch. The catch may have a shape determined by its relationship with a corresponding portion of the cylindrical housing 31, and may be also realized by not only a projection such as the flange 92 but also a recess, a slip preventing surface and so on.

The liquid feeding mechanism 30 is assembled, centered around the cylindrical housing 31. The liquid feeding mechanism 30 comprises a pushing operating portion (an operating member) 40, a fixed angle rotating mechanism (or a linear/rotary motion converting mechanism) 50, and a returning mechanism 70 when it is broadly divided.

The pushing operating portion 40 comprises the above-mentioned knob 41. The knob 41 is fixed on a knob shaft 42, and the knob shaft 42 is held in the knob guiding member 43 such that it can freely advance or retreat (so as to be axially movable) and in an unrotatable state (a state where the rotation is prevented). The user pushes the knob 41 with his or her hand.

Axial motion of the knob 41 (movement more than a first predetermined stroke) is converted into predetermined-angle rotation in one direction (this is referred to as forward rotation) of a fixed angle rotating member 51 and the screw 60 by the fixed angle rotating mechanism 50. The screw 60 and the fixed angle rotating member 51 are together rotated and are coupled to (engage with) each other so as to be axially movable.

The screw 60 has a screw formed on its circumferential surface. Amale screw 33A is formed on an inner circumferential surface of the tip end portion 33 of the cylindrical housing 31, and the screw 60 is screwed into the male screw 33A. That is, the screw 60 is held in the cylindrical housing 31 by the portion 33 so as to be rotatable and such that it can freely advance or retreat by the screwing.

The screw 60 is rotated in the forward direction through a predetermined angle, thereby to advance by a second stroke (a predetermined value).

A tip end member 61 is provided at a tip end of the screw 60. The tip end member 61 is made of a hard material such as plastics, and its tip end is in a hemispherical shape, that is, is rounded. When the screw 60 advances, the tip end member 61 presses the piston 93 in the aqueous medication ampoule 90. The screw 60 advances while rotating. Since the tip end of the tip end member 61 is rounded, the rotation of the screw 60 does not constitute an obstacle to pressing the piston 93.

When the knob 41 is pushed by more than the first stroke (the amount of movement thereof is determined because the knob 41 abuts against an end surface 43A (serving as a stopper) of the knob guiding member 43), its linear motion is converted into a predetermined-angle rotation (60° in the present embodiment) of the screw 60 by the fixed angle rotating mechanism 50 (the details will be described later). Therefore, the screw 60 advances by a second stroke. The piston 93 in the aqueous medication ampoule 90 advances in the ampoule 90 by the same stroke as the second stroke, and a aqueous medication with which the ampoule 90 (a portion between the piston 93 and the tip end of the ampoule) is filled is jetted with great force from the minute hole 93 at the tip end. Since the amount of movement of the piston 93 is constant, and the inner diameter of the ampoule 90 is also constant along the length thereof (the ampoule is basically cylindrical), the amount of aqueous medication jetted from the ampoule 90 is also constant.

Although an inner surface at the tip end of the ampoule 90 is tapered in a conical shape, the tip end surface of the piston 93 is also tapered in entirely the same shape (in a conical shape). Therefore, the piston 93 is finally right along the inner surface at the tip end of the ampoule 90, so that the aqueous medication is entirely jetted.

When the knob 41 is moved (pushed) by 8 mm, for example, the piston 93 is moved by 0.3 mm. The amount of movement of the knob 41 is regulated by the existence of the stopper 43A. When the user merely presses or pushes the knob 41 until the knob 41 abuts against the stopper 43A, therefore, the piston 93 is properly moved by a predetermined stroke. Accordingly, a predetermined amount of aqueous medication is accurately supplied from the ampoule 90. It is preferable that the first stroke is considerably longer than the second stroke, as in the present embodiment.

The cylindrical housing 31 is formed with a flange 32, and a screw 32A is formed at the front of the flange 32. The screw 32A of the cylindrical housing 31 is screwed into the male screw in the mounting member 5 until the flange 32 abuts against the mounting member 5. Consequently, the cylindrical housing 31 is fixed at its accurate position.

Fig. 5 illustrates a state where the tip end of the screw 60 significantly enters the ampoule 90. The screw 60 can be rotated in the opposite direction by a returning mechanism 70, described later, to retreat into the cylindrical housing 31. When a new aqueous medication ampoule 90 is mounted on the cylindrical housing 31, the tip end of the screw 60 retreats into approximately the same position as that of the tip end of the cylindrical housing 31.

A battery 3 and a printed circuit board 2 on which electric circuits (a controller), described later, is assembled are provided in the device housing 1.

A portion near the atomizing casing 10 on the upper surface of the device housing 1 is formed in a projecting shape, and a power switch 85 is embedded in this portion. The power switch 85 comprises a button 85A projecting upward. When the cylindrical housing 31 is set in a posture in its used state with the aqueous medication ampoule 90 mounted on the cylindrical housing 31, the button 85A in the power switch 85 is pressed by the ampoule 90, so that the power switch 85 is turned on.

Mainly referring to Fig. 6, the device housing 1 is formed with a boss 4, and a male screw is formed on an inner circumferential surface of the boss 4. A horn holder 23 is in an approximately cylindrical shape, and a screw is formed on its outer circumferential surface. This screw is screwed into the male screw of the boss 4, whereby the horn holder 23 is fixed. The horn holder 23 has an upper wall, and an opening 23A is formed in the upper wall.

An ultrasonic vibrator horn 20 is circular in lateral cross section, and is formed such that its upper half has a small diameter and its lower half has a large diameter. A horn supporting ring 24 is fitted in an annular groove formed on a circumferential surface of an upper part of the large-diameter lower half of the horn 20. The ring 24 is fixed by being interposed between the shoulder of the upper wall of the holder 23 and an inner cylinder 25 tightly fitted in the holder 23. Consequently, the horn 20 is supported on the holder 23 by means of only the ring 24, and is basically spaced apart from the holder 23 in a portion other than the ring 24. A seal ring (an O ring or a packing) 26 is provided between the shoulder of the large-diameter lower half of the horn 20 and the upper wall of the holder 23.

A piezoelectric element (a piezoelectric vibrator) 21 is fixed (for example, bonded) to a lower surface of the horn 20. The piezoelectric element 21 is interposed between electrodes 22 from above and below. A voltage having an ultrasonic frequency is applied to the electrodes 22 by a driving circuit (a controller), described later. Consequently, the piezoelectric element 21 ultrasonically vibrates, and the vibration is transmitted to the horn 20.

An atomizing casing (an atomizing cap) 10 is detachably mounted (fitted) on the boss 4. The atomizing casing 10 comprises a mounting portion 10A in its lower half which is fitted on the boss 4 and an atomizing portion 10B in its upper half. The casing 10 is integrally formed of plastics, for example.

The atomizing portion 10B of the casing 10 is basically in a cylindrical shape, and has a bottom wall. The bottom wall is formed with a hole 11 into which the upper half 20A of the horn 20 loosely enters with a clearance therebeteen. When the casing 10 is mounted at a proper position, an upper end of the small-diameter upper half 20A of the horn 20 projects upward from the position of the hole 11, passing through the hole 11 of the atomizing portion 10B. Description is hereinafter made of the positional relationship in a state where the casing 10 is properly mounted, and the cylindrical housing 31 on which the aqueous medication ampoule 90 is mounted is set in its used state. An inner space of the atomizing portion 10B of the casing 10 is referred to as an atomizing chamber 10C.

A mesh plate 15 is placed on an upper end surface of the horn 20 in the atomizing chamber 10C. The mesh plate 15 is a thin plate-shaped member having a lot of minute (very small) holes formed thereon. The mesh plate 15 is for promoting the atomization of the liquid by the ultrasonic vibration.

The mesh plate 15 is pressed from above by the pressing spring 14, and tightly brought into contact with to the upper end surface of the horn 20. The pressing spring 14 is a coil spring which increases in diameter upward. An inner circumferential surface of the atomizing portion 10B is formed so as to be depressed in its upper part, and a spring receiving member 13 is fitted therein from above. As a result, a spring receiving groove 12 is formed below the spring receiving member 13, and a portion having the largest diameter of the pressing spring 14 is engaged therewith.

A liquid feeding (supplying) hole 16 is formed on the circumferential wall of the atomizing portion 10B at a height nearly corresponding to an upper part of the upper half 20A of the horn 20 at a position which the tip end of the ampoule 90 faces. A liquid feeding (supplying) pipe 17 is tightly inserted into the liquid feeding hole 16. An inner end of the liquid feeding pipe 17 is in close proximity to the upper part of the upper half 20A of the horn 20, and is at a position just below the mesh plate 15 . An inner end (a tip end) of the liquid feeding pipe 17 is cut obliquely (in a direction nearer to the horn downward). The center of the liquid feeding hole 16 and the center of the minute hole 91 at the tip end of the ampoule 90 coincide with each other. There is a small clearance at the position of the liquid feeding hole 16 and the minute hole 91 between an outer surface of the peripheral wall of the atomizing portion 10B and a tip end surface of the ampoule 90. The inner diameters of the liquid feeding hole 16 and the liquid feeding pipe 17 are slightly larger than the inner diameter of the minute hole 91.

A plate 18 is placed at the bottom of the atomizing portion 10B. The plate 18 has a hole at its center, and is slightly bent downward around the hole. A portion, bent downward, of the plate 18 enters the hole 11 of the atomizing portion 10B. There is also a clearance between an inner peripheral surface of the hole at the center of the plate 18 (the portion bent downward) and the upper half 20A of the horn 20, and the plate 18 is not brought into contact with the horn 20. This is for preventing the vibration of the horn 20 from being attenuated. The plate 18 is for preventing the aqueous medication fed into the atomizing chamber 10C from dropping downward through the hole 11. When the amount of the aqueous medication to be fed is small, the plate is not necessarily required.

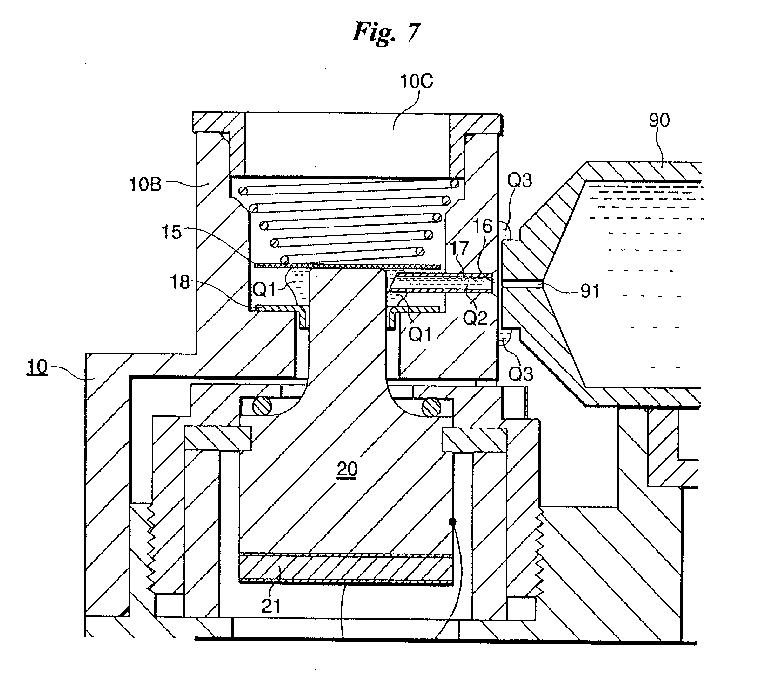

The knob 41 is pushed, as described above, whereby the aqueous medication in the aqueous medication ampoule 90 is jetted from the minute hole 91 at its tip end, and is fed into the atomizing chamber 10C through the liquid feeding hole 16 and the liquid feeding pipe 17. Fig. 7 shows a state where the aqueous medication is injected into the atomizing chamber 10C.

A tip end of the liquid feeding pipe 17 reaches the vicinity of the horn 20, and the amount of aqueous medication to be injected into the atomizing chamber 10C is small. Accordingly, the fed aqueous medication adheres (attaches) to the vicinity of the upper half (particularly, its upper part) 20A of the horn 20 and the vicinity of a lower surface of the mesh plate 15 by surface tension (indicated by Q1). The aqueous medication easily adheres because the tip end of the liquid feeding pipe 17 is oblique. The aqueous medication also exists in the liquid feeding pipe 17 (indicated by Q2). The aqueous medication which cannot entirely enter the liquid feeding pipe 17, although it is jetted from the minute hole 91 of the aqueous medication ampoule 90 (if it exists) is held in a clearance existing between the outer surface of the peripheral wall of the atomizing portion 10B and the tip end of the ampoule 90 (indicated by Q3).

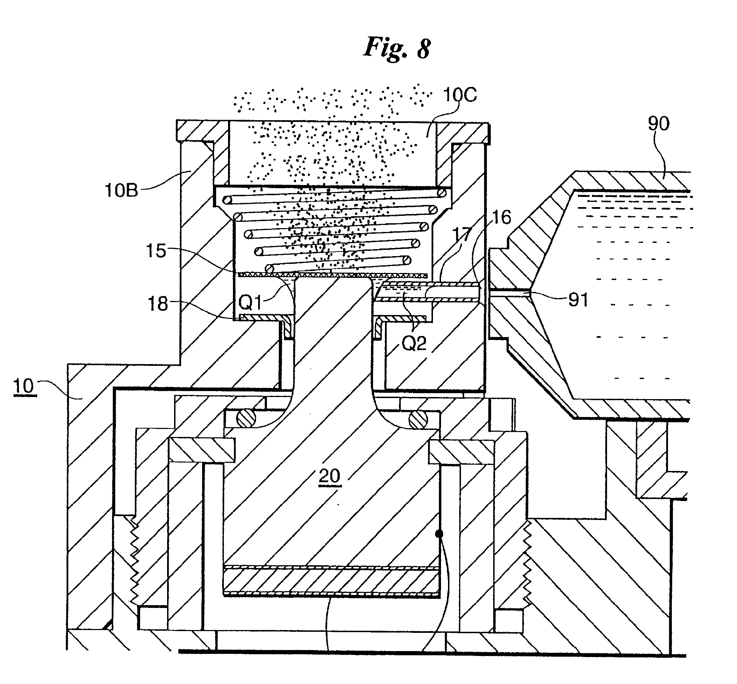

When the aqueous medication is fed (supplied) into the atomizing chamber 10C from the ampoule 90, the ultrasonic vibration of the horn 20 (the piezoelectric element 21) is started (or is strengthened) immediately (as described later), and the aqueous medication which adheres to the upper part of the horn 20 is atomized from an upper opening of the atomizing chamber 10C, as shown in Fig. 8. The liquid feeding pipe 17 and the liquid feeding hole 16 are made narrow in diameter to such an extent that the aqueous medication in their inner parts is sucked out (a capillary action or phenomenon) by the influence of the atomization. The aqueous medication Q3 held in the clearance between the outer surface of the peripheral wall of the atomizing chamber 10B and the tip end surface of the ampoule 90 is also sucked into the liquid feeding pipe 17, and is finally atomized. Consequently, almost all of the aqueous medication fed into the atomizing chamber 10C or its vicinity is atomized in a short time.

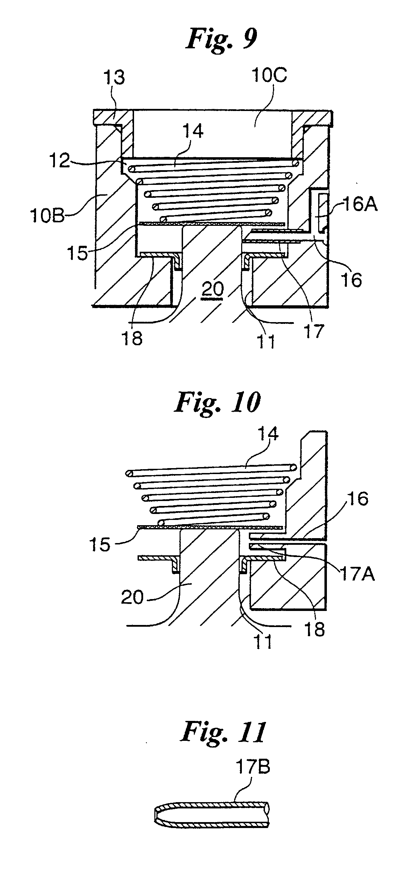

Fig.9 illustrates a modified example. A liquid reservoir 16A communicating with a liquid feeding hole 16 is formed in a peripheral wall of an atomizing chamber 10B. The liquid reservoir 16A opens toward the air. A aqueous medication which cannot entirely enter a liquid feeding pipe 17, although it is jetted from a minute hole 91 of the ampoule 90, is held in the liquid reservoir 16A. When atomization is started by ultrasonic vibration, the aqueous medication in the liquid reservoir 16A is sucked in through the liquid feeding pipe 17. When the liquid reservoir 16A is formed, a clearance between the peripheral wall of the atomizing portion 10B and a tip end of the ampoule 90 is not necessarily required.

Fig. 10 illustrates a mode in which a part of the peripheral wall of the atomizing portion 10B is projected in a direction nearer to the horn 20 (a projection 17A) instead of providing the liquid feeding pipe 17, and the liquid feeding hole 16 is extended into the projection 17A. The mode is effective for a case where the atomizing portion 10B is miniaturized.

As shown in Fig. 11, the tip end of the liquid feeding pipe 17B is tapered, so that a force to suck the aqueous medication, which is caused by the atomization, is strengthened.

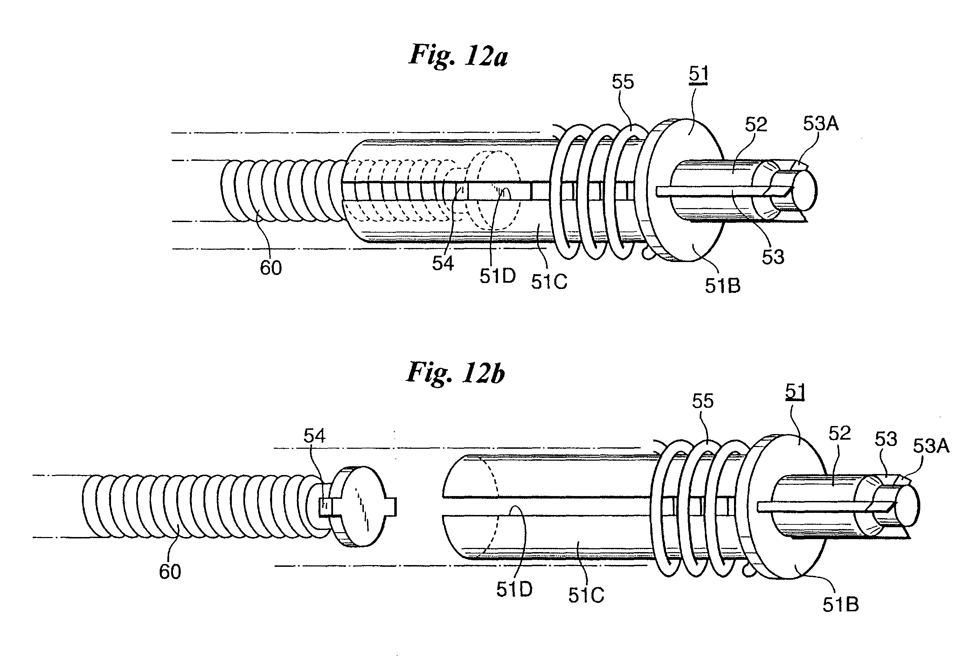

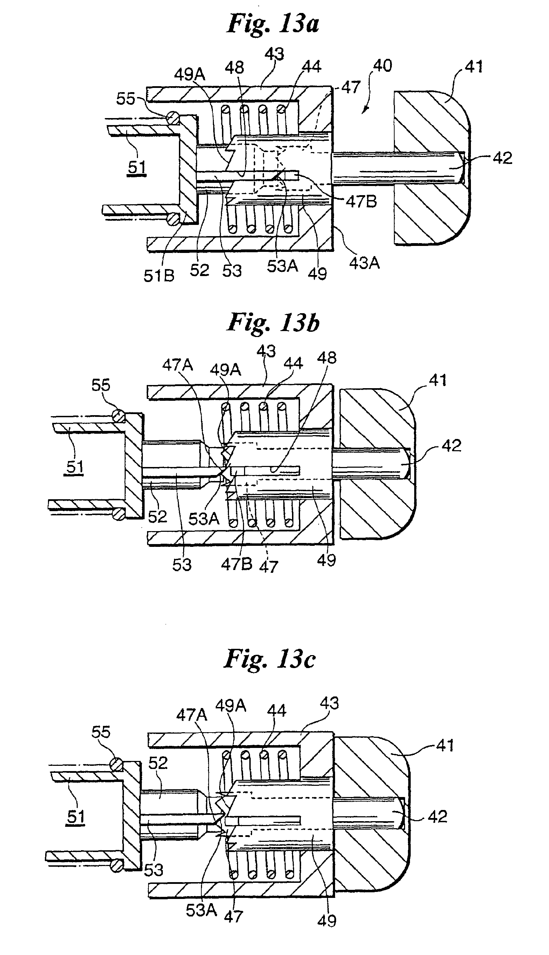

Referring to Figs. 12a to 12b and 13a to 13h, description is made of the pushing operating portion 40 and the fixed angle rotating mechanism 50.

Referring to Figs. 5 and 12, the fixed angle rotating member 51 comprises a flange 51B, a mounting projection 51A projecting from the flange 51B, and a cylindrical portion 51C fixed to the flange 51A on the opposite side of the mounting projection 51A. A screw 60 is accommodated so as to be movable (slidable) in the cylindrical portion 51C. Two slits 51D are formed on the cylindrical portion 51C. Engaging projections 54 formed on the screw 60 enter the slits 51D. Consequently, the fixed angle rotating member 51 and the screw 60 are together rotated, and are axially slidable from each other.

An inner portion of the cylindrical housing 31 is so formed that the inner diameter thereof is slightly larger over a longer range than a range in which the flange 51B of the fixed angle rotating member 51 is moved. A pressing (compressed) spring (a return spring 55) is provided between a distal end (a step 39) of the portion having a large inner diameter and the flange 51B. The fixed angle rotating member 51 is always urged in the direction forward the knob 41 by the spring 55.

A rotating member 52 is tightly fixed to the mounting projection 51A of the fixed angle rotating member 51. The rotating member 52 comprises a large-diameter portion at its base end and a small-diameter portion at its tip end, and three sliding fingers 53 are provided at equal angle intervals on their peripheral surfaces. The sliding finger 53 is formed with a slope 53A at the tip end thereof.

In Figs. 13a to 13h, only one of the sliding fingers 53 is illustrated in order to make the description easy to understand.

The knob guiding member 43 is a cylindrical member having a bottom. The knob guiding member 43 is mounted so as to cover the outer end of the cylindrical housing 31, and is coupled to the cylindrical housing 31 by a lock pin 74, described later. A lock spring 44 is provided between an outer end surface of the cylindrical housing 31 and the knob guiding member 43, and the knob guiding member 43 is always urged toward the knob 41.

The knob guiding member 43 has a cam cylinder 49 formed toward inside on its bottom wall. A shaft hole 43B through which a knob shaft 42 passes is formed on the bottom wall of the knob guiding member 43. The inside of the cam cylinder 49 communicates with the shaft hole 43B.

Slits 48 which the sliding fingers 53 enter so as to be slidable are formed at three positions, corresponding to the sliding fingers 53, of the cam cylinder 49. Two oblique cam surfaces 49A are formed between the slits 48 on a top end surface of the cam cylinder 49. The adjacent oblique cam surfaces 49A are separated by a raised wall axially directed.

A pressing member 47 is fixedly provided at an inner end of the knob shaft 42. Cam surfaces 47A, which are slopes inclined at an angle which coincides with slope of the sliding finger 53, are formed on an end surface of the pressing member 47. Rotation preventing projections 47B which enter the slits 48 are formed on a peripheral surface of the pressing member 47. Consequently, the knob shaft 42 moves along its axis in a state where the rotation thereof is prevented. The shaft hole 43B of the knob guiding member 43 has a step, and the pressing member 47 abuts against the step. Accordingly, the pressing member 47 is prevented from coming off the shaft hole 43B, and a position projecting outward of the knob shaft 42 (which is pressed by the spring 55) is regulated (limitted).

In a state shown in Fig. 13a, the sliding finger 53 enters the slit 48 of the cam cylinder 49. When the knob 41 is pushed by the user, the cam surface 47A of the pressing member 47 presses the slope 53A of the sliding finger 53 against a force of the spring 55, as shown in Fig. 13b.

At a position where the sliding finger 53 comes off the slit 48, the slope 53A of the sliding finger 53 slightly slides along the cam surface 47A of the pressing member 57 (because it is pressed by the spring 55), as shown in Fig. 13c. At this time, the rotating member 52 (i.e., the fixed angle rotating member 51 and the screw 60) rotates slightly (through an angle of approximately 10°).

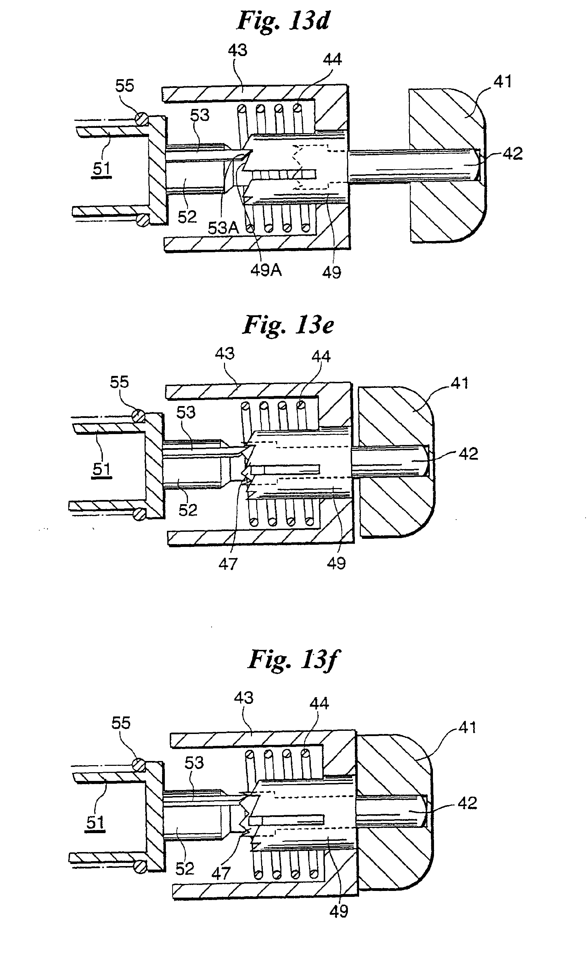

When the user stops pushing the knob 41, and takes his or her hand off the knob 41, the knob 41 is returned outward. A tip end of the sliding finger 53 of the rotating member 52 which slightly rotates is put on the oblique cam surface 49A of the cam cylinder 49, and slides along the cam surface 49A (because it is pressed by the spring 55), as shown in Fig. 13d. As a result, the rotating member 52 (i.e., the fixed angle rotating member 51 and the screw 60) rotates greatly (through an angle of approximately 50°). Consequently, the screw 60 is rotated through an angle of a total of 60°.

When the knob 41 is pushed again, the pressing member 47 presses the sliding finger 53 against the force of the spring 55 again, as shown in Fig. 13e, to separate the tip end of the sliding finger 53 from the oblique cam surfaces 49A of the cam cylinder 49. When the tip end of the sliding finger 53 is pressed beyond the height of the raised wall which is the boundary of the oblique cam surfaces 49A, the sliding finger 53 slightly slides on the cam surface 47A of the pressing member 47, and rotates slightly (through an angle of 10°), as shown in Fig. 13f.

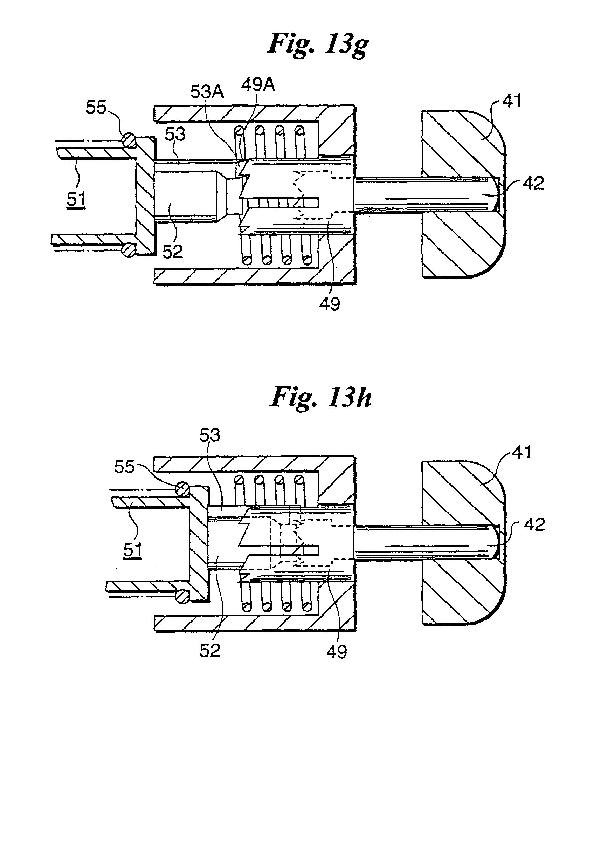

When the pressing of the knob is released, the sliding finger 53 is put on the subsequent (adjacent) oblique cam surface 49A of the cam cylinder 49 (consequently, the rotating member 52 rotates greatly (through an angle of 50°)), as shown in Fig. 13g. Finally, the sliding finger 53 enters the subsequent slit 48 of the cam cylinder 49, as shown in Fig. 13h. This is the same as the state shown in Fig. 13a (while the screw 60 rotates through an angle of 120°) .

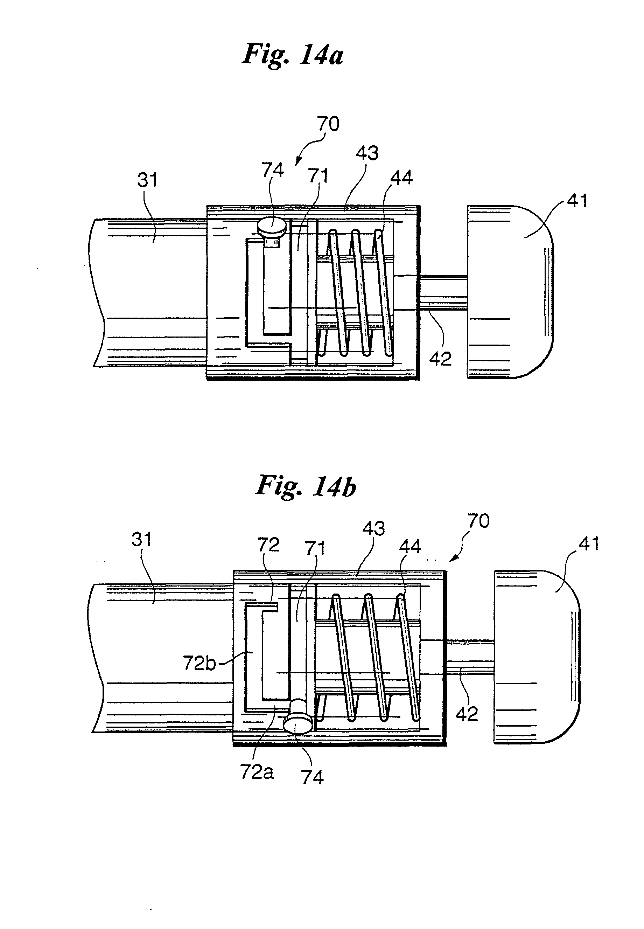

Figs. 14a and 14b illustrate the returning mechanism 70. In the figures, the lock guiding member 43 is drawn as a transparent one so that an annular groove 71 and a lock groove 72 which are formed on the cylindrical housing 31 are seen.

The annular groove 71 and the lock groove 72 are formed on an outer peripheral surface of the outer end portion of the cylindrical housing 31. The lock groove 72 connects with the annular groove 71 through a groove 72a axially extending from the annular groove 71 and a groove 72b parallel to the annular groove 71. The lock groove 72 is formed in the axial direction toward the annular groove 71, and is terminated without connecting with the annular groove 72.

The lock pin 74 penetrates the lock guiding member 43, and its tip end enters the annular groove 71 or the lock groove 72 so as to be freely movable therethrough.

Generally, the lock pin 74 enters the lock groove 72, as shown in Fig. 14a. Consequently, the lock guiding member 43 is integral with the cylindrical housing 31. In this state, it is possible to feed the aqueous medication by pressing the knob 41, described above.

When the lock pin 74 is inserted into the annular groove 71, as shown in Fig. 14b, by shifting the relative position between the lock guiding member 43 and the cylindrical housing 31, the lock guiding member 43 is rotatable with respect to the cylindrical housing 31. When the lock guiding member 43 is rotated in the reverse direction (in the direction in which the screw 60 is caused to retreat) in this state, its rotating force is transmitted to the fixed angle rotating member 51 through the engagement of the sliding finger 53 in the fixed angle rotating mechanism 50, to rotate the screw 60 in the reverse direction to retreat. When the tip end of the screw 60 reaches approximately the same position as the tip end of the cylindrical housing 31, it is possible to remove the aqueous medication ampoule 90 which is emptied (or almost emptied) and to mount a new aqueous medication ampoule.

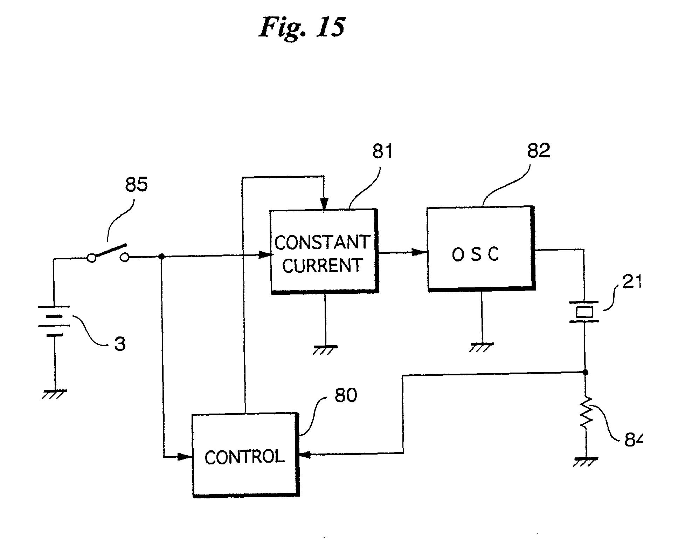

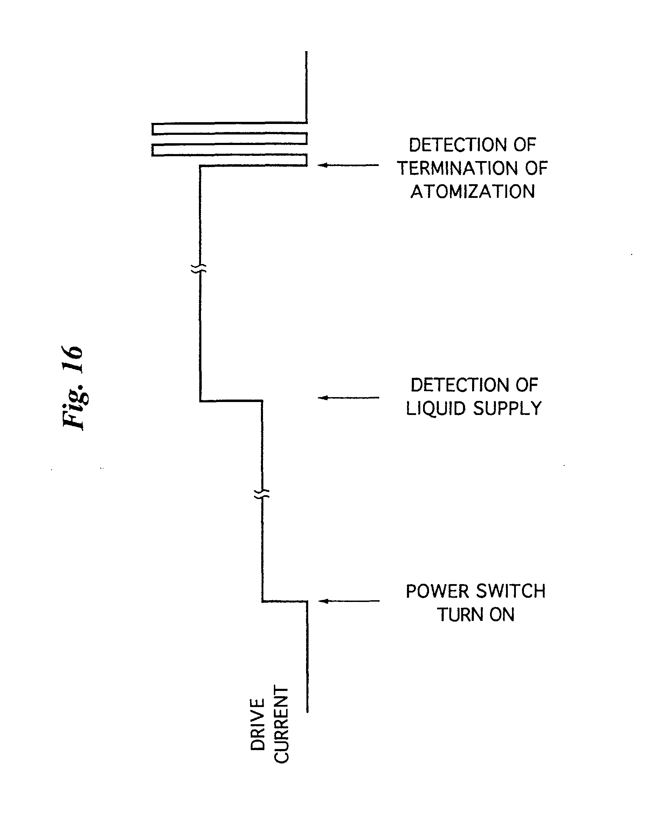

Fig. 15 illustrates an example of an atomization controller (control circuit) provided on the circuit board 2 in the device housing 1. Fig. 16 is a waveform diagram showing the operations thereof.

When the cylindrical housing 31 on which the aqueous medication ampoule 90 is mounted is brought into the use state, the switch 85 is turned on. Accordingly, the power of the battery 3 is supplied to the circuit.

A constant current circuit 81 can switch its output current in three stages. When the switch 85 is turned on, the constant current circuit 81 outputs the lowest current under the control of a control circuit 80. An oscillation circuit 82 starts to oscillate, and the piezoelectric element 21 ultrasonically vibrates. The ultrasonic vibration at this time is weak. Consequently, the battery 3 can be prevented from being consumed.

When the aqueous medication is supplied into the atomizing chamber 10C by pushing the knob 41, the supplied aqueous medication adheres to an upper part of the horn 20. Accordingly, the impedance of the horn 20 which weakly vibrates is changed. The change is detected as the change in a voltage or the like which appears on a resistor 84 connected to the piezoelectric element 21 or other element.

When the control circuit 80 detects the feed of the aqueous medication, it controls the constant current circuit 81 so as to set an output of the constant current circuit 81 to a normal value. The ultrasonic vibration is continued by sufficient power to atomize the fed aqueous medication. Consequently, nearly all of the fed aqueous medication is atomized.

An atomization start switch may be provided, to produce ultrasonic vibration for atomization by input from the switch. In this case, the necessity of weak ultrasonic vibration based on the turn-on of the switch 85 is dispensed with.

When the atomization of the aqueous medication is almost terminated, the control circuit 80 detects the termination of the atomization on the basis of the change in the impedance again. The termination of the atomization may be detected on the basis of the elapsed time (for example, two seconds) from the start of the atomization.

Thereafter, the control circuit 80 controls the constant current circuit 81 such that stronger ultrasonic vibration is generated in a pulse fasion at short time intervals (for example, 0.1 seconds). Preferably, stronger pulse-shaped vibration is performed a plurality of times.

Consequently, a small amount of aqueous medication remaining on the mesh plate 15, between the mesh plate 15 and the upper end surface of the horn 20, or the like is atomized, thereby making it possible to make the amount of aqueous medication remaining without being atomized very small.

Another embodiment of the present invention will be described.

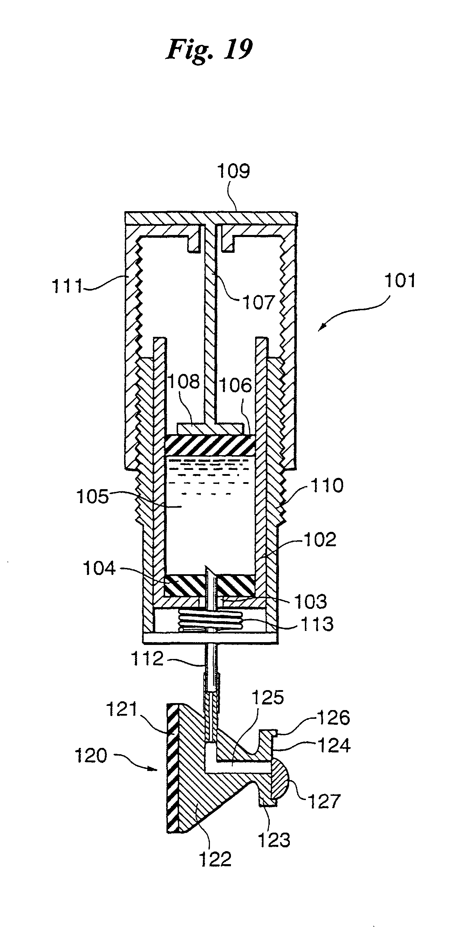

Fig. 19 is a cross-sectional view showing the structures of a liquid feeding portion and an atomizing portion in an ultrasonic inhaler.

A liquid feeding portion 101 comprises an inner accommodating portion 110 accommodating a aqueous medication bottle 102 and an outer accommodating portion 111 combined therewith. An outer surface of the inner accommodating portion 110 and an inner surface of the outer accommodating portion 111 are threaded, and are meshed with each other.

An aqueous medication bottle 102 has a hole 103 formed on its bottom surface, and an elastic member 104 made of rubber or the like for sealing the hole 103 is accommodated in its inner part. An aqueous medication 105 in the aqueous medication bottle 102 is held in the bottle 102 water-tightly by a piston 106.

A hollow liquid feeding pipe 112 in the shape of a needle whose tip end is pointed is provided at a position facing the hole 103 of the aqueous medication bottle 102 on a bottom surface of the inner accommodating portion 110. Further, a coil spring 113 for urging the aqueous medication bottle 102 upward is provided between a bottom surface of the aqueous medication bottle 102 and the bottom surface of the inner accommodating portion 110.

The outer accommodating portion 111 comprises a piston pressing bar 107 having a piston pressing portion 108 at its lower end and a button 109 at its upper end.

An atomizing portion 120 comprises a horn (an amplitude converter) 122 of a conical shape. A piezoelectric vibrator 121 is fixed to a large end surface of the horn 122, and a resonance plate 123 in a disk shape is fixed to a small end surface thereof. A liquid feeding path 125 connected to the liquid feeding pipe 112 is formed inside the horn 122. The liquid feeding path 125 is bent in an L shape inside the horn 122, and opens at the resonance plate 123. A projection 126 is formed at an outer peripheral edge of the resonance plate 123, to prevent an aqueous medication 127 on an atomizing surface 124 from leaking and dropping easily by vibration or the like.

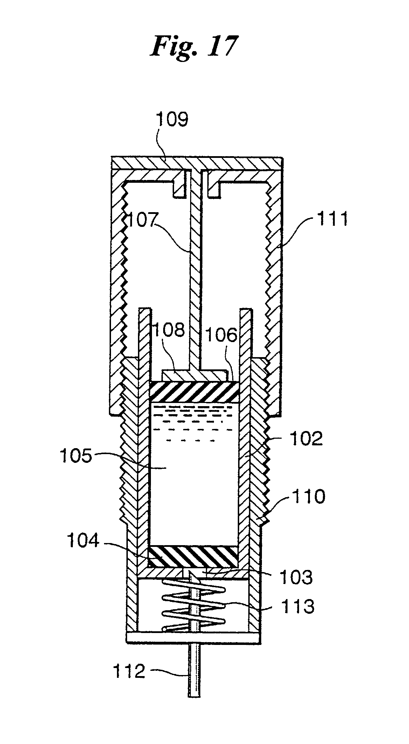

Operations for feeding the aqueous medication 105 corresponding to one prescription to the atomizing surface 124 of the atomizing portion 120 from the aqueous medication bottle 102 in a state where the aqueous medication 105 is accommodated in the aqueous medication bottle 102 will be described with reference to Figs. 17 to 20. In the figures excluding Fig. 19, the illustration of the atomizing portion is omitted.

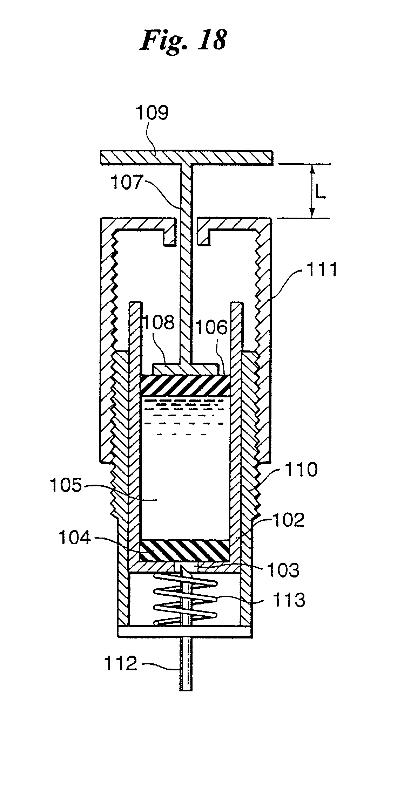

Fig. 17 illustrates an initial state, which is a state where the button 109 and an upper end surface of the outer accommodating portion 111 are brought into contact with each other. The outer accommodating portion 111 is rotated and is moved in a direction nearer to the inner accommodating portion 110 to provide a suitable distance L between the button 109 and the upper end surface of the outer accommodating portion 111, as shown in Fig. 18, thereby setting the amount of feed of an aqueous medication corresponding to one prescription.

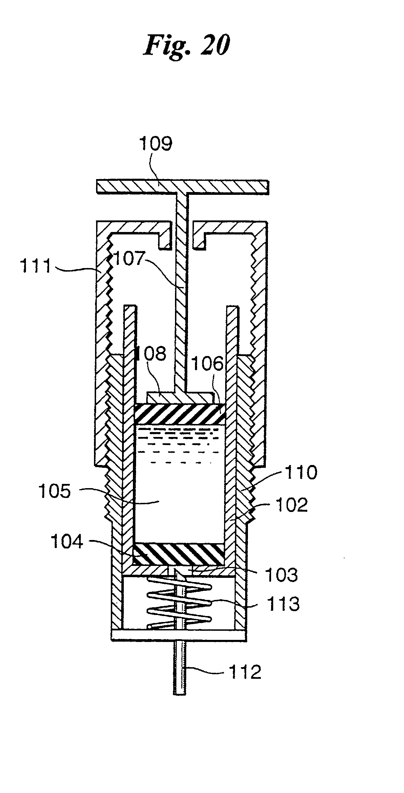

When a user presses the button 109 downward, the piston 106 is pressed downward through the piston pressing bar 107. The whole of the aqueous medication bottle 102 is first moved downward against a force of the coil spring 113. By the movement, a tip end of the liquid feeding pipe 112 breaks (penetrates) through the elastic member 104 through the hole 103. Accordingly, the liquid feeding pipe 112 communicates with the inside of the aqueous medication bottle 102. As shown in Fig.19, the aqueous medication corresponding to one stroke of the piston pressing bar 107 is ejected as a aqueous medication corresponding to one prescription, and is fed into the atomizing surface 124 through the liquid feeding path 125. When the user takes his or her fingers off the button 109, the aqueous medication bottle 102 is moved upward, as shown in Fig. 20, by an urging force of the coil spring 113. A tip end of the liquid feeding pipe 112 comes off the elastic member 104, so that the aqueous medication bottle 102 enters its original sealed state. When the needle of the liquid feeding pipe 112 comes off the elastic member 104, a hole of the elastic member 104 is closed, so that the aqueous medication does not leak outward.

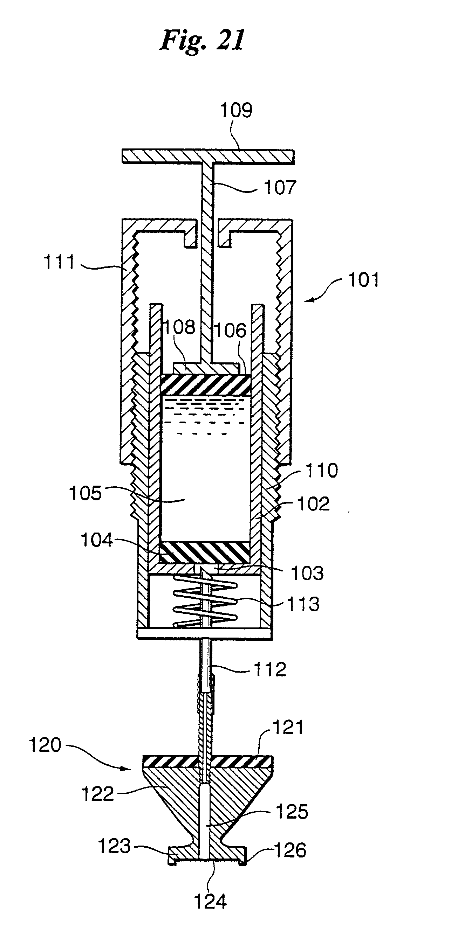

The liquid feeding path 125 of the ultrasonic vibrator in the atomizing portion 120 may be in a linear shape, as shown in Fig. 21. This makes the fabrication of the ultrasonic vibrator easier.

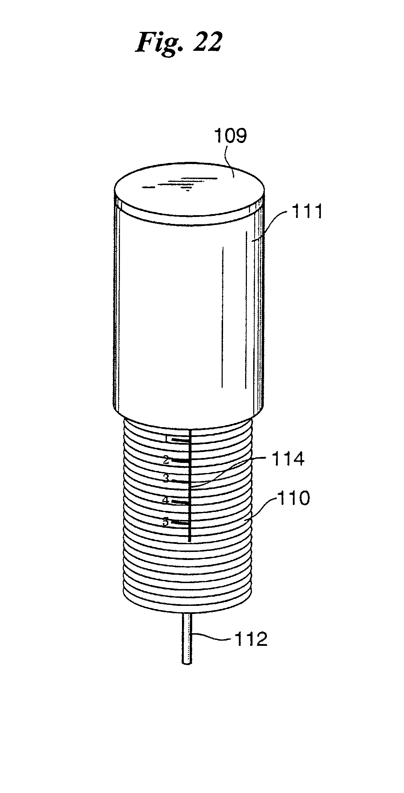

In order to set the amount of feed of a aqueous medication corresponding to one prescription, a scale 114 can be also provided on an outer surface of the inner accommodating portion 110 as shown in Fig. 22.

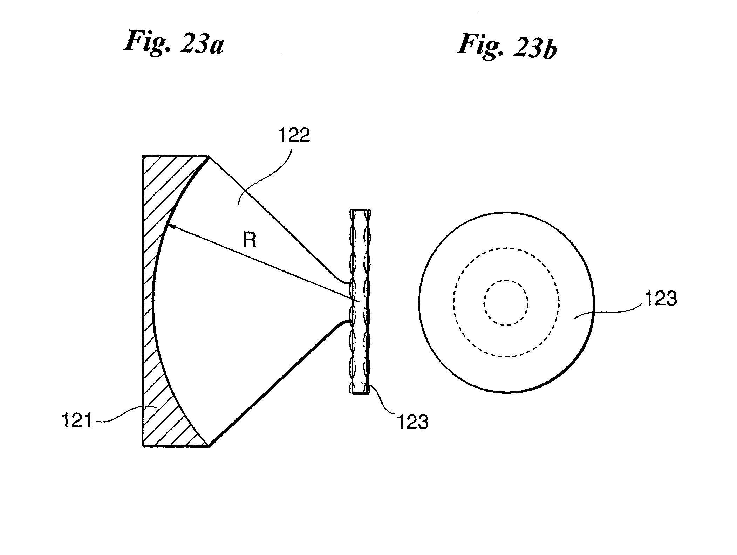

In order to improve the vibration efficiency, that is, the atomization efficiency, an ultrasonic vibrator as shown in Figs. 23a and 23b may be used.

An end surface having a large area of the horn 122 is brought into the shape of a spherical surface, and a surface of the piezoelectric vibrator (piezoelectric element) 121 is also brought into the shape of a spherical surface so as to be along the spherical surface such that the center of the radius of curvature R of the spherical surface coincides with the center of the resonance plate 123. Consequently, vibration generated in the piezoelectric vibrator 121 is concentrated on the resonance plate 123, the vibration displacement of the resonance plate 123 is increased, thereby improving the vibration efficiency.

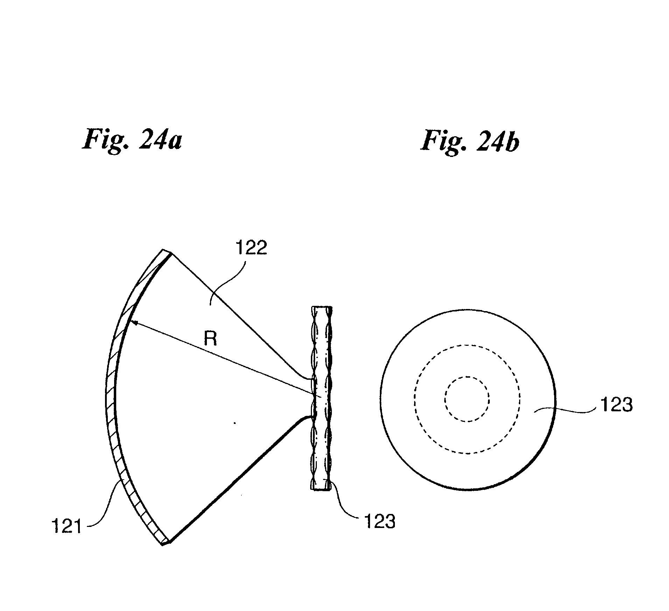

As shown in Figs. 24a and 24b, the piezoelectric vibrator 121 may be along the spherical surface of the horn 122.

In the ultrasonic vibrators, if the diameter of an end surface having a small area of the horn 122 coincides with the node inner diameter of the resonance plate 123, and the diameter and thickness of the resonance plate 123 are selected such that the number of circular nodes is more than two, vibration displacement in a megahertz zone is obtained at high efficiency. For this purpose, the radius of curvature may be so set as to be an odd multiple of one-fourth of a wavelength which is determined by a resonance frequency.

## Claims

1. A liquid feeding mechanism for atomizing a liquid, comprising:
a cylindrical housing having an end on which a liquid bottle having a movable piston disposed therein is mountable;
a pushing operating member which is pressed to be moved by more than a first predetermined stroke and is returned to its original position;
a fixed angle rotating mechanism for converting movement by more than the first stroke of said pushing operating member into forward rotation of a predetermined angle; and
a screw which is held in said cylindrical housing so as to be rotatable by screwing and is disposed so as to advance by a second predetermined stroke when it is rotated in a forward direction through the predetermined angle by said fixed angle rotating mechanism to press the piston in said liquid bottle.

2. The liquid feeding mechanism according to claim 1, wherein said first stroke is longer than said second stroke.

3. The liquid feeding mechanism according to claim 1 or 2, further comprising a stopper for regulating the amount of movement of said pushing operating member.

4. The liquid feeding mechanism according to any one of claims 1 to 3, wherein said screw is provided with a portion projecting outward at a tip end thereof, and a tip end of said portion is rounded.

5. The liquid feeding mechanism according to any one of claims 1 to 4, further comprising a returning mechanism which engages with said fixed angle rotating mechanism for rotating said fixed angle rotating mechanism in a reverse direction to cause said screw to retreat.

6. The liquid feeding mechanism according to claim 1, wherein
said pushing operating member comprises a knob guiding member which is provided so as to be rotatable at the other end of said cylindrical housing, a knob shaft which is provided so as to be axially movable in a state where the rotation thereof is prevented by the knob guiding member, and a knob which is provided at an outer end of the knob shaft, and
said fixed angle rotating mechanism comprises a fixed angle rotating member which engages with said screw so as to be rotated together with the screw and so as to be movable along the axis direction of the screw, a sliding finger which is fixed to the fixed angle rotating member, extends along the knob shaft, and has a slope formed at a tip end thereof, a return spring for urging said fixed angle rotating member toward said knob guide, a cam cylinder which is provided inside said knob guiding member, and has an oblique cam surface which the tip end of said sliding finger brought into contact with and slides by a force of said return spring to rotate said fixed angle rotating member, and a pressing member which is provided at an inner end of said knob shaft, and has a cam surface for pressing said sliding finger against the force of said return spring in contact with the slope at the tip end of said sliding finger when said knob shaft is pressed inward through said knob to release the engagement of said sliding finger with said cam cylinder and slightly rotating said fixed angle rotating member to put the tip end of said sliding finger on the subsequent oblique cam surface of said cam cylinder.

7. The liquid feeding mechanism according to claim 6, wherein said returning member comprises said knob guiding member, said knob guiding member is of a cylindrical shape and is provided so as to cover said other end portion of said cylindrical housing, an annular groove and a lock groove connecting therewith are formed on a peripheral surface of said other end portion of said cylindrical housing, an inner end of a lock pin provided so as to penetrate a peripheral wall of said knob guiding member enters said annular groove or said lock groove so as to be movable, and a lock spring for urging said knob guiding member outward is provided between said other end of said cylindrical housing and said knob guiding member.

8. A liquid feeding mechanism for atomizing a liquid, comprising:
a cylindrical housing having an end on which a liquid bottle having a movable piston disposed therein is mountable;
an operating member which is moved by a manual operation and is returned to its original position;
a fixed angle rotating mechanism for converting movement of said operating member into forward rotation of a predetermined angle; and
a screw which is held in said cylindrical housing so as to be rotatable by screwing and is disposed so as to advance by a predetermined stroke when it is rotated in a forward direction through the predetermined angle by said fixed angle rotating mechanism to press the piston in said liquid bottle.

9. A liquid bottle, which has a minute hole formed at a tip end thereof, opens at a distal end thereof, has a movable piston disposed therein, and is provided with a catch used to mount the liquid bottle on a liquid feeding mechanism for pressing said piston and for resisting a force for pressing said piston.

10. The liquid bottle according to claim 9, wherein said piston is formed of an elastic member, and is provided with a hard receiving member which receives a pressing force from said liquid feeding mechanism.

11. The liquid bottle according to claim 9 or 10, wherein said catch is a flange formed at the distal end.

12. The liquid bottle according to any one of claims 9 to 11, further comprising a cap for covering said tip end including said minute hole.

13. An atomizing mechanism comprising an atomizing chamber in which at least a part of an ultrasonic vibrator is provided, wherein a liquid feeding hole is formed on a wall of the atomizing chamber, a liquid feeding pipe is provided from the liquid feeding hole toward the inside of the atomizing chamber, a tip end of the liquid feeding pipe reaches the vicinity of said ultrasonic vibrator, and a liquid buffer exists in a part of said liquid feeding hole or the liquid feeding pipe.

14. The atomizing mechanism according to claim 13, wherein said liquid feeding pipe is a hole communicating with said liquid feeding hole and formed in a projection formed integrally with the wall of said atomizing chamber so as to project toward the inside of said atomizing chamber.

15. The atomizing mechanism according to claim 13 or 14, wherein said liquid buffer is a clearance between a tip end of the liquid bottle having the minute hole for feeding the liquid into the atomizing chamber through said liquid feeding hole formed therein and an outer surface of the wall of the atomizing chamber.

16. The atomizing mechanism according to claim 13 or 14, wherein said liquid buffer is a liquid reservoir formed halfway in said liquid feeding hole or the liquid feeding pipe and opening toward the air.

17. The atomizing mechanism according to any one of claims 13 to 16, wherein a tip end of said liquid feeding pipe is obliquely cut.

18. The atomizing mechanism according to any one of claims 13 to 17, wherein said ultrasonic vibrator comprises a horn and a mesh plate provided on an upper end surface of the horn.

19. An atomizing mechanism comprising:
an atomizing casing having a cylindrical atomizing chamber having a hole formed on its lower surface;
a horn, a part of which projects into said atomizing chamber from said hole of said casing, driven by ultrasonic vibration;
a mesh plate disposed on an upper end surface of said horn;
a pressing spring for urging said mesh plate in the direction in which the mesh plate is brought into contact with the upper end surface of said horn;
a liquid feeding pipe penetrating the wall of said atomizing chamber, and introduced into the vicinity of the upper end portion of said horn; and
a plate placed on a bottom surface of said atomizing chamber and having a hole formed therein having such an inner diameter as to hold a clearance between the hole and a peripheral surface of said horn.

20. The atomizing mechanism according to any one of claims 13 to 18, further comprising a driving control circuit for stopping the driving of said ultrasonic vibrator once at the time point when the atomization of the liquid fed into the atomizing chamber through said liquid feeding pipe is almost terminated, and then driving said ultrasonic vibrator once or intermittently a plurality of times in a short time.

21. The atomizing mechanism according to claim 20, wherein the ultrasonic vibrator is more strongly vibrated in said short time.

22. In a device for feeding a predetermined amount of liquid to an ultrasonic vibrator to atomize the liquid by the vibration of the ultrasonic vibrator, an atomization controller comprising:
means for detecting the time point where the atomization of the liquid fed into the ultrasonic vibrator is almost terminated; and
first control means for carrying out control so as to stop the driving of the ultrasonic vibrator once in response to the detection of the time point where the atomization of the liquid is almost terminated and to drive the ultrasonic vibrator once or intermittently a plurality of times in a short time after an elapse of a short time since the driving was stopped.

23. The atomization controller according to claim 22, further comprising
means for detecting the feed of the liquid to the ultrasonic vibrator in a state where the ultrasonic vibrator is weakly vibrated, and
second control means for carrying out control so as to drive the ultrasonic vibrator by normal power in response to the detection of the feed of the liquid.

24. The atomization controller according to claim 23, wherein the first control means carries out control so as to drive the ultrasonic vibrator more strongly than the second control means.

25. The atomization controller according to claim 23, further comprising third control means for carrying out control so as to weakly vibrate the ultrasonic vibrator in response to the turn-on of a power switch.

26. A liquid atomizer, wherein an atomizing chamber is provided in a part of a device housing, a liquid feeding mechanism is provided in the other part of the device housing, at least a part of a ultrasonic vibrator is disposed inside said atomizing chamber, a liquid feeding hole is provided in a part of a wall of said atomizing chamber, a liquid bottle having a minute hole formed at its tip end and having a movable piston provided therein is mountable on one end of said liquid feeding mechanism, said liquid feeding mechanism can be positioned such that the minute hole of the liquid bottle mounted on said liquid feeding mechanism and the liquid feeding hole formed in said atomizing chamber coincide with each other, and said liquid feeding mechanism has a manual operating member and a pressing mechanism for causing the piston in the mounted liquid bottle to advance by a predetermined stroke when the manual operating member is operated.

27. The liquid atomizer according to claim 26, wherein a small clearance exists between the tip end, at which the minute hole is formed, of said liquid bottle and an outer surface of the wall, on which the liquid feeding hole is formed, of said atomizing chamber at a position where the minute hole of said liquid bottle and the liquid feeding hole of said atomizing chamber coincide with each other.

28. The liquid atomizer according to claim 26 or 27, wherein said ultrasonic vibrator comprises an ultrasonic vibrator horn at least a part of which is disposed in said atomizing chamber and a mesh plate disposed so as to be brought into contact with an upper end of the horn by being pressed by a spring, the liquid feeding pipe extends from said liquid feeding hole toward the vicinity of the upper end portion of said horn.

29. The liquid atomizer according to claim 28, wherein the tip end of said liquid feeding pipe is obliquely cut.

30. The liquid atomizer according to any one of claims 26 to 29, wherein said device housing is provided with a mounting member for detachably mounting said liquid feeding mechanism.

31. The liquid atomizer according to claim 30, wherein said mounting member is movable.

32. The liquid atomizer according to any one of claims 26 to 31, wherein said device housing is provided with a positioning member for said liquid feeding mechanism.

33. The liquid atomizer according to claim 26, comprising a cover for covering at least its part provided with said atomizing chamber.

34. The liquid atomizer according to claim 26, wherein said liquid feeding mechanism comprises a fixing member for fixing said liquid bottle.

35. The liquid atomizer according to claim 26, wherein
said liquid feeding mechanism comprises a cylindrical housing having an end on which said liquid bottle is mountable,
said manual operating member is moved by a manual operation and is returned to its original position, and
said pressing mechanism comprises a fixed angle rotating mechanism for converting movement of said manual operating member into forward rotation of a predetermined angle, and a screw which is held in said cylindrical housing so as to be rotatable by screwing and is disposed so as to advance by a predetermined stroke when it is rotated in a forward direction through the predetermined angle by said fixed angle rotating mechanism to press the piston in said liquid bottle.

36. The liquid atomizer according to claim 35, wherein said liquid feeding mechanism further comprises a returning mechanism which engages with said fixed angle rotating mechanism and rotates in a reverse direction said fixed angle rotating mechanism to cause said screw to retreat.

37. The liquid atomizer according to claim 26, wherein said device housing has a driving control circuit provided therein for stopping the driving of said ultrasonic vibrator once at the time point where the atomization of the liquid fed into the atomizing chamber through said liquid feeding pipe is almost terminated, and then driving said ultrasonic vibrator once or intermittently a plurality of times in a short time.

38. The liquid atomizer according to claim 26, wherein said device housing has a controller provided therein, said controller comprising
means for detecting the time point where the atomization of the liquid fed into the ultrasonic vibrator is almost terminated, and
control means for carrying out control so as to stop the driving of the ultrasonic vibrator once in response to the detection of the time point where the atomization of the liquid is almost terminated, and then drive the ultrasonic vibrator once or intermittently a plurality of times in a short time after an elapse of a short time since the driving was stopped.

39. An inhaler using a liquid bottle having a hole provided at its one end, the hole being sealed with an elastic member provided in the bottle, having a movable piston provided at the other end, and filled with a liquid, comprising:
inner and outer accommodating portions which are fitted to each other so as to be movable, the inner accommodating portion accommodating the liquid bottle so as to be movable, and comprising a hollow needle and a returning member, and the outer accommodating portion comprising a member for pressing the piston in the liquid bottle,
said inner accommodating portion being moved when the piston in the liquid bottle accommodated in the inner accommodating portion is pressed by said pressing member manually operated, said needle penetrating said elastic member so that the liquid in the liquid bottle is fed into the ultrasonic vibrator through said needle, and said inner accommodating portion being returned by said returning member when a pressing force of said pressing member is removed.

40. An ultrasonic vibrator comprising a horn in an approximately conical shape, a piezoelectric element provided at the base of the horn, and a resonance plate in a disk shape integrally formed at a tip end of the horn, in which
an end surface at the base of the horn which is provided with said piezoelectric element is in a spherical shape, and the piezoelectric element is provided along the end surface in the spherical shape.
